# EUROPEAN PATENT APPLICATION

(11) **EP 4 699 523 A1**
(43) Date of publication of application: **25.02.2026**
(21) Application number: 24854313.4
(22) Date of filing: 09.07.2024
(51) Int. Cl.: A61B 5/01, A61B 5/00

(54) **ELECTRONIC DEVICE AND METHOD FOR MEASURING TEMPERATURE BY USING SAME**

(30) Priority: 11.08.2023 KR 20230105703; 07.09.2023 KR 20230118938
(71) Applicant: Samsung Electronics Co., Ltd., Suwon-si, Gyeonggi-do 16677 (KR)
(72) Inventor: JO, Seongwook, Suwon-si, Gyeonggi-do 16677 (KR); KWON, Hyoujoo, Suwon-si, Gyeonggi-do 16677 (KR); PARK, Hyuncheol, Suwon-si, Gyeonggi-do 16677 (KR); YOO, Soohan, Suwon-si, Gyeonggi-do 16677 (KR); LEE, Seungwon, Suwon-si, Gyeonggi-do 16677 (KR); JUNG, Hyunjun, Suwon-si, Gyeonggi-do 16677 (KR); CHO, Shinhee, Suwon-si, Gyeonggi-do 16677 (KR)
(74) Representative: Gulde & Partner
(86) International application number: PCT/KR2024/009793
(87) International publication number: WO 2025/037761

(57) **Abstract**

A wearable electronic device is provided. The wearable electronic device includes a housing including a first surface, and a second surface positioned to be opposite to the first surface and configured to come into contact with a part of a body when the wearable electronic device is worn, a temperature sensor disposed in an internal space of the housing and configured to measure a temperature of an object adjacent to the first surface, an inertial sensor disposed in the internal space, memory storing one or more computer programs, and one or more processors communicatively connected to the temperature sensor, the inertial sensor, and the memory, wherein the one or more computer programs include computer-executable instructions that, when executed by the one or more processors, cause the wearable electronic device to detect a motion of the wearable electronic device by using the inertial sensor, when a first condition corresponding to the detected motion is satisfied, activate the temperature sensor, identify the object on a basis of the detected motion, and measure the temperature of the identified object by using the activated temperature sensor.

## Description

### [Technical Field]

The disclosure relates to an electronic device and a temperature measurement method using the same.

### [Background Art]

With recent technological advancements, electronic devices have gradually changed in shapes while departing from uniform rectangular shapes. For example, the electronic devices have been gradually developed toward wearable electronic devices that can be worn on parts of human bodies in order to improve portability or user accessibility. The wearable electronic devices may include an electronic device that can be worn on a part of the human body (e.g., hand, wrist, finger, head, neck, or ear). The wearable electronic devices can be worn on a user's finger and provide various user experiences and beneficial functions.

The above information is presented as background information only to assist with an understanding of the disclosure. No determination has been made, and no assertion is made, as to whether any of the above-might be applicable as prior art with regard to the disclosure.

### [Disclosure of Invention]

### [Technical Problem]

An electronic device (e.g., a wearable electronic device or a ring-shaped (e.g., shape, shape, type) portable electronic device) may have a structure in which a plurality of constituent elements (e.g., a temperature sensor, an inertial sensor (e.g., an acceleration sensor or a gyro sensor), a photoplethysmogram (PPG) sensor, and a photoplethysmography sensor) is disposed in a narrow internal space. The electronic device may be at least partially mounted on and fixed to a user's finger. When the electronic device is worn on the finger, the electronic device may be kept at least partially in contact with the user's skin.

The wearable electronic device, particularly, a ring-type wearable electronic device configured to be worn on the user's finger may be connected to an external electronic device (e.g., a smartphone) in a wireless manner. The wearable electronic device may acquire the user's bio-information (e.g., temperature information or body temperature) by means of at least one sensor module (e.g., a temperature sensor) and provide the user's bio-information to the external electronic device. In addition, the wearable electronic device may provide the user with information (e.g., a notification signal or a notification message), which is provided from the external electronic device, in a visual, auditory, or tactile manner.

The electronic device may include a temperature sensor provided in an internal space and configured to measure a temperature. For example, the temperature sensor may include a contactless temperature sensor that may measure a temperature of an object (e.g., a part of a human body or an item) without being in physical contact with the object. The temperature sensor may acquire radiant rays (radiation) (e.g., electromagnetic waves, radio waves, or infrared energy emitted from the object) based on infrared rays (IRs) generated (e.g., emitted) from a surface of the object, and the temperature sensor may measure the temperature of the object on the basis of the acquired radiant rays.

According to an embodiment of the disclosure, in the electronic device, the temperature sensor (e.g., the contactless temperature sensor) may be at least partially disposed in the internal space and have a sensing area formed in a predetermined direction (e.g., an outward direction) of an outer surface (e.g., an outer surface or an external environment). The electronic device may use temperature sensor to measure the temperature of the object at least partially positioned in the sensing area. For example, the electronic device may be worn in a state in which the electronic device is at least partially mounted on the user's finger and measure the temperature of the object based on the sensing area formed in a direction in which the palm is directed.

Aspects of the disclosure are to address at least the above-mentioned problems and/or disadvantages and to provide at least the advantages described below. Accordingly, an aspect of the disclosure is to provide a ring-shaped electronic device configured to be mounted on a user's human body (e.g., a finger) and measure a temperature of an object by using a temperature sensor.

Additional aspects will be set forth in part in the description which follows and, in part, will be apparent from the description, or may be learned by practice of the presented embodiments.

### [Solution to Problem]

In accordance with an aspect of the disclosure, a wearable electronic device is provided. The wearable electronic device includes a housing including a first surface, and a second surface positioned to be opposite to the first surface and configured to come into contact with a part of a body when the wearable electronic device is worn, a temperature sensor disposed in an internal space of the housing and configured to measure a temperature of an object adjacent to the first surface, an inertial sensor disposed in the internal space, memory storing one or more computer programs, and one or more processors communicatively connected to the temperature sensor, the inertial sensor, and the memory, wherein the one or more computer programs include computer-executable instructions that, when executed by the one or more processors, cause the wearable electronic device to detect a motion of the wearable electronic device by using the inertial sensor, when the first condition corresponding to the detected motion is satisfied, activate the temperature sensor, identify the object on a basis of the detected motion, and measure the temperature of the identified object by using the activated temperature sensor.

In accordance with another aspect of the disclosure, a method of measuring a temperature by using a wearable electronic device is provided. The method includes detecting a motion of the wearable electronic device by using an inertial sensor disposed in an internal space of a housing, activating a temperature sensor disposed in the internal space of the housing in response to a situation in which a first condition corresponding to the detected motion is satisfied, identifying an object on a basis of the detected motion, and measuring a temperature of the identified object by using the activated temperature sensor.

In accordance with another aspect of the disclosure, one or more non-transitory computer-readable storage media storing one or more computer programs including computer-executable instructions that, when executed by one or more processors of a wearable electronic device, cause the wearable electronic device to perform operations are provided. The operations include detecting the motion of the wearable electronic device by using the inertial sensor disposed in the internal space of the housing, activating a temperature sensor disposed in the internal space of the housing in response to the situation in which the first condition corresponding to the detected motion is satisfied, identifying the object on a basis of the detected motion, and measuring the temperature of the identified object by using the activated temperature sensor.

### [Advantageous Effects of Invention]

According to the embodiment, the electronic device includes a ring-shaped wearable electronic device that may be at least partially worn on a part of the human body (e.g., the finger) of the user. For example, the user may measure a temperature of a specific object anytime, anywhere, easily and conveniently while wearing the wearable electronic device in daily life. According to the embodiment, the electronic device may measure the temperature of the object and provide the user with a notification signal related to the measured temperature, which may improve the convenience in identifying the temperature of the object.

According to the embodiment, the electronic device includes the inertial sensor configured to detect the motion of the electronic device. In case that the motion of the electronic device satisfies a trigger condition (e.g., a first condition for activating the temperature sensor), the electronic device may at least partially activate the temperature sensor. According to the embodiment, the electronic device may determine whether to activate the temperature sensor on the basis of the motion, which may reduce electric power consumption required to operate the temperature sensor. The efficiency of the battery of the electronic device may be improved.

According to the embodiment, the electronic device may distinguish and recognize the motions of the electronic device by using the inertial sensor. For example, when the motion of the electronic device is matched with a first gesture, the electronic device may acquire a first temperature of a first object (e.g., a first user) corresponding to the first gesture. For example, when the motion of the electronic device is matched with a second gesture, the electronic device may acquire a second temperature of a second object (e.g., a second user) corresponding to the second gesture. The electronic device may independently distinguish and manage the first temperature of the first object (e.g., the user, i.e., the wearer) and the second temperature of the second object (e.g., another person). The electronic device may more conveniently manage the plurality of objects and the temperatures of the objects.

Other aspects, advantages, and salient features of the disclosure will become apparent to those skilled in the art from the following detailed description, which, taken in conjunction with the annexed drawings, discloses various embodiments of the disclosure.

### [Brief Description of Drawings]

The above and other aspects, features, and advantages of certain embodiments of the disclosure will be more apparent from the following description taken in conjunction with the accompanying drawings, in which:
FIG. 1 is a block diagram of an electronic device in a network environment according to an embodiment of the disclosure;
FIG. 2 is a cross-sectional view of the electronic device according to an embodiment of the disclosure;
FIG. 3 is a block diagram illustrating the electronic device according to an embodiment of the disclosure;
FIG. 4 is a flowchart illustrating a method of measuring a temperature of an object by the electronic device according to an embodiment of the disclosure;
FIG. 5 is an exemplified view illustrating an embodiment in which an inertial sensor is used to identify a motion of the electronic device according to an embodiment of the disclosure;
FIG. 6A is an exemplified view illustrating an embodiment in which a first temperature of a first object is measured in accordance with a first gesture according to an embodiment of the disclosure;
FIG. 6B is an exemplified view illustrating an embodiment in which a second temperature of a second object is measured in accordance with a second gesture according to an embodiment of the disclosure;
FIG. 6C is an exemplified view illustrating an embodiment in which a third temperature of a third object is measured in accordance with a third gesture according to an embodiment of the disclosure; And
FIG. 6D is an exemplified view illustrating an embodiment in which a preset function is performed in response to the detection of a preset gesture according to an embodiment of the disclosure.

Throughout the drawings, it should be noted that like reference numbers are used to depict the same or similar elements, features, and structures.

### [Mode for the Invention]

The following description with reference to the accompanying drawings is provided to assist in a comprehensive understanding of various embodiments of the disclosure as defined by the claims and their equivalents. It includes various specific details to assist in that understanding, but these are to be regarded as merely exemplary. Accordingly, those of ordinary skill in the art will recognize that various changes and modifications of the various embodiments described herein can be made without departing from the scope and spirit of the disclosure. In addition, descriptions of well-known functions and constitution may be omitted for clarity and conciseness.

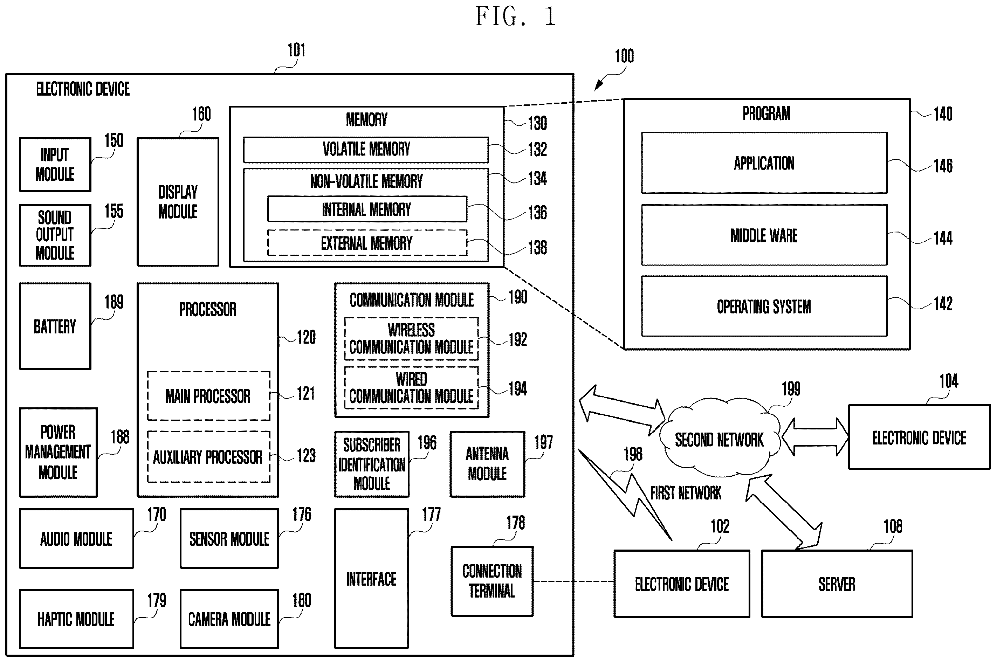

FIG. 1 is a block diagram illustrating an example electronic device 101 in a network environment 100 according to an embodiment of the disclosure. Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to an embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, an HDMI connector, a USB connector, an SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a fifth generation (5G) network, after a fourth generation (4G) network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the millimeter wave (mmWave) band) to achieve, e.g., a high data transmission rate. The wireless communication module 192 may support various technologies for securing performance on a high-frequency band, such as, e.g., beamforming, massive multipleinput and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element including a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, an RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band. For example, the plurality of antennas may include a patch array antenna and/or a dipole array antenna.

At least some of the above-described components may be coupled mutually and communicate signals (e.g., commands or data) therebetween via an interperipheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In an embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

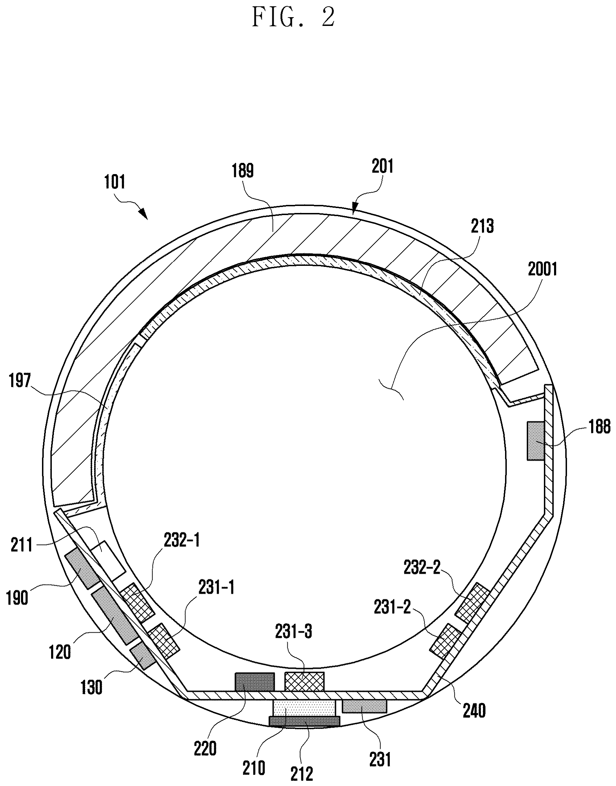

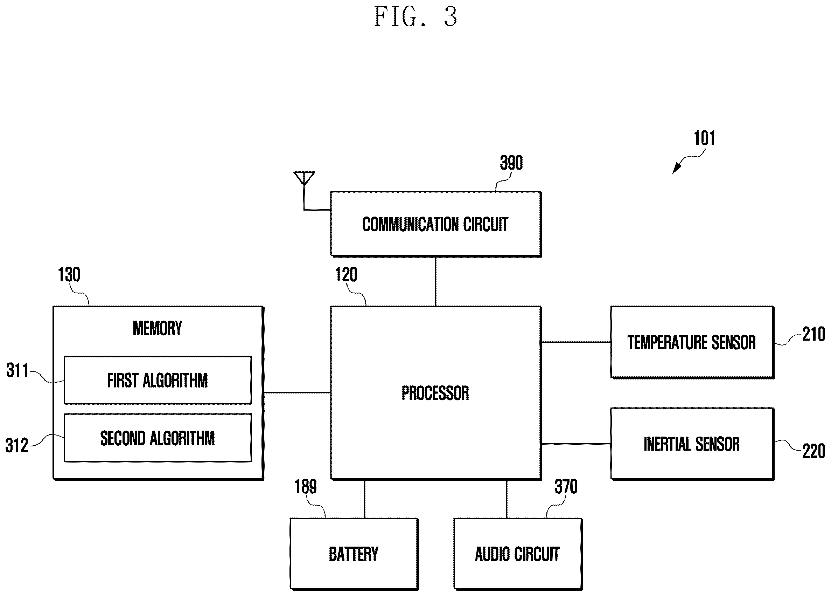

FIG. 2 is a cross-sectional view of the electronic device according to an embodiment of the disclosure. FIG. 3 is a block diagram illustrating the electronic device according to an embodiment of the disclosure. FIG. 3 is a block diagram of the electronic device including the constituent elements illustrated in FIG. 2.

Referring to FIGS. 2 and 3, the electronic device 101 (e.g., the electronic device 101 in FIG. 1) may be at least partially similar to the electronic device 101 in FIG. 1 or further include other embodiments of the electronic device. For example, the electronic device 101 may include a wearable electronic device capable of being worn on a part of a human body (e.g., a finger).

In the description of the electronic device 101 (e.g., the wearable electronic device) according to the disclosure, a ring-type (e.g., ring-shape) wearable electronic device worn on a user's finger is illustrated and described. However, the disclosure is not limited thereto. For example, it will be apparent to those skilled in the art that the embodiments of the disclosure may be applied to a bracelet-type wearable electronic device, an open-ring-type electronic device opened partially, or a curved or non-curved electronic device.

Referring to FIGS. 2 and 3, the electronic device 101 (e.g., the wearable electronic device) may include an annular housing 201 including an opening 2001 therein. In the embodiment, the housing 201 may include a first surface (e.g., an outer surface or an outer ring housing) configured to be exposed to an external environment in a state in which the electronic device 101 is worn on a part of the human body (e.g., the finger), and a second surface (e.g., an inner surface or an inner ring housing) positioned to be opposite to the first surface and configured to be at least partially in contact with the finger's skin in the worn state. The opening 2001 may be sized so that the user's finger is fitted with the opening 2001.

Referring to FIG. 2, the electronic device 101 may include at least one electric element disposed in an internal space of the housing 201. At least one electric element may include at least one constituent element among at least one biometric sensor (e.g., temperature sensors 210 and 211 and/or PPG sensors 231-1, 231-2, 231-3, 232-1, and 232-2) disposed to detect bio-information (e.g., a body temperature) of an object (e.g., a user) through at least a part (e.g., the first surface or the second surface) of the housing 201, an inertial sensor 220 (e.g., a 3-axis sensor, a 6-axis sensor, an acceleration sensor, or a gyro sensor) configured to detect a motion of the electronic device 101, a processor (e.g., the processor 120 in FIG. 1), memory (e.g., the memory 130 in FIG. 1), a communication circuit (e.g., the communication module 190 in FIG. 1), a substrate 240 (e.g., a printed circuit board (PCB) or a flexible printed circuit board (FPCB)), a power management module (e.g., the power management module 188 in FIG. 1), and/or a battery (e.g., the battery 189 in FIG. 1).

At least one biometric sensor (e.g., the temperature sensors 210 and 211 and/or the photoplethysmogram (PPG) sensors 231-1, 231-2, 231-3, 232-1, and 232-2) (e.g., a photoplethysmogram sensor or a photoplethysmography sensor) may acquire bio-information of the object (e.g., the user). The temperature sensors 210 and 211 may include a first temperature sensor 210 corresponding to a contactless temperature sensor, and a second temperature sensor 211 corresponding to a contact temperature sensor. For example, the contactless temperature sensor (e.g., the first temperature sensor 210) may acquire radiant rays (radiation) (e.g., electromagnetic waves, radio waves, or infrared energy emitted from the object) based on infrared rays (IRs) generated (e.g., emitted) from a surface of the object, and the temperature sensor may measure the temperature of the object on the basis of the acquired radiant rays. The contactless temperature sensor may infer a temperature by measuring thermal radiation emitted from the object. The contactless temperature sensor may measure the temperature of the object on the basis of infrared emissivity for the object in a state in which the contactless temperature sensor is not in physical contact with the object (e.g., a part of the user's body). For example, the contact temperature sensor (e.g., the second temperature sensor 211) may measure the temperature on the basis of heat of the object in a state in which the contact temperature sensor is in physical contact with the object (e.g., a part of the user's body). The contact temperature sensor (e.g., a thermocouple temperature sensor) may include two conductors (e.g., conductive members) and identify a change in electromotive force (emf, electromotive force) between the two conductors in a state in which one conductor is in physical contact with the object. The contact temperature sensor may measure the temperature of the object on the basis of the change in electromotive force caused by the physical contact with the object.

The temperature sensors 210 and 211 may include the first temperature sensor 210 (e.g., the contactless temperature sensor) disposed along the first surface (e.g., the outer surface) of the housing 201, and the second temperature sensor 211 (e.g., the contact temperature sensor) disposed along the second surface (e.g., the inner surface) of the housing 201. The PPG sensors 231-1, 231-2, 231-3, 232-1, and 232-2 may measure pulse waves of a second object (e.g., the user or the wearer) positioned in a second direction, along the second surface (e.g., the inner surface) of the housing 201. For example, the PPG sensors may include light-emitting-PPG sensors 231-1, 231-2, and 231-3 configured to emit light, and light-receiving-PPG sensors 232-1 and 232-2 configured to receive light. For example, an arrangement angle and an arrangement position of the first temperature sensor 210 may be determined to conform to a situation in which a temperature of a counterpart in a state in which the user opens the palm. As another example, an arrangement angle, an arrangement interval, and an arrangement distance of the PPG sensor may be configured in advance in order to measure a more accurate temperature of the object in the state in which the electronic device 101 is worn on the user's finger. For example, an arrangement angle, an arrangement interval, and an arrangement distance of the PPG sensor may be determined on the basis of the physical contact with the object in accordance with a situation in which the temperature of the object (e.g., the user) is measured.

The first temperature sensor 210 (e.g., the contactless temperature sensor) disposed on the first surface (e.g., the outer surface) of the housing 201 may acquire a first temperature of a first object (e.g., a counterpart or another person) positioned in a first direction with respect to the first surface. For example, the processor 120 may acquire radiant rays from the first object (e.g., the counterpart or another person) by means of an infrared transmission lens 212 and calculate the first temperature of the first object on the basis of the acquired radiant rays. The second temperature sensor 211 (e.g., the contact temperature sensor) disposed on the second surface (e.g., the inner surface) of the housing 201 may measure a second temperature of a second object (e.g., the user or the wearer) positioned in a second direction with respect to the second surface. For example, the processor 120 may measure the temperature of the second object (e.g., the user) by means of the second temperature sensor 211 (e.g., the contact temperature sensor) disposed to be in physical contact with a part of the human body (e.g., the finger) in the state in which the electronic device 101 is mounted on the user's finger.

In case that the wearer measures his/her temperature by using the first temperature sensor 210, the processor 120 may calculate the temperature of the wearer on the basis of the first temperature measured by the first temperature sensor 210 and the second temperature measured by the second temperature sensor 211. For example, the processor 120 may at least partially apply a correction value, which corresponds to the second temperature, to the first temperature on the basis of the first temperature in a state in which the first temperature is configured as a main temperature, and the second temperature is configured as a sub-temperature. In the situation in which the plurality of temperature sensors (e.g., the first temperature sensor 210 and the second temperature sensor 211) is used to measure the temperature of the same first object, the processor 120 may calculate the temperature of the first object on the basis of a first-first temperature measured by the first temperature sensor 210 and the first-second temperature measured by the second temperature sensor 211.

In the method of measuring the temperature of the object (e.g., the counterpart or another person) by using the electronic device 101, the temperature sensor may be defined as the first temperature sensor 210 (e.g., the contactless temperature sensor). The temperature sensor 210 illustrated in FIG. 3 may be defined as the first temperature sensor 210 corresponding to the contactless temperature sensor.

The PPG sensors 231-1, 231-2, 231-3, 232-1, and 232-2 (e.g., the photoplethysmogram sensor or the photoplethysmography sensor) disposed on the second surface (e.g., the inner surface) of the housing 201 may identify a flow of blood (e.g., a blood flow) by using light. For example, when the blood flows along blood vessels, a blood flow rate finely changes. The PPG sensor may measure the pulse waves (plethysmogram (PTG)) of the user (e.g., the user wearing the electronic device 101 on the finger) on the basis of the amount of change in blood flow rates. The PPG sensors may include the light-emitting-PPG sensors 231-1, 231-2, and 231-3 configured to emit light, and the light-receiving-PPG sensors 232-1 and 232-2 configured to receive light. For example, the light emitted from the light-emitting-PPG sensors 231-1, 231-2, and 231-3 is at least partially reflected by the object, and the light-receiving-PPG sensors 232-1 and 232-2 may receive at least a part of the reflected light. The processor 120 may measure the user's pulse waves on the basis of the received light. The PPG sensors 231-1, 231-2, 231-3, 232-1, and 232-2 may be controlled by a PPG control module 231. According to the embodiment, the PPG sensor is not limited to a particular type (e.g., a reflective type or a transmissive type) and to a blood flow measurement method based on a particular method. For example, the PPG sensor may be implemented as a reflective type, a transmissive type, and other types.

The inertial sensor 220 may include at least one of a 3-axis sensor, a 6-axis sensor, an acceleration sensor, and/or a gyro sensor. An arrangement angle, an arrangement posture, and an arrangement position of the inertial sensor 220 may be determined based on a preset particular posture (e.g., an upright posture) of the electronic device 101. The inertial sensor 220 may acquire coordinate information (e.g., gesture information or movement information) in accordance with a posture, a position, and a motion of the electronic device 101. The processor 120 may detect various motions (e.g., a finger motion, a first gesture, a second gesture, and a third gesture of the user wearing the electronic device 101) of the electronic device by means of the inertial sensor 220. For example, the processor 120 may select and recognize the first gesture in which the user touches his/her forehead with the hand, the second gesture in which the user touches another person's forehead with the hand, and the third gesture in which the user surrounds and grasps a particular object with the hand. The processor 120 may identify the objects (e.g., persons or items) corresponding to the gestures and apply algorithms corresponding to the objects at the time of measuring the temperatures of the identified objects. For example, in case that the first object is a person, the processor 120 may measure the first temperature of the first object on the basis of a first algorithm stored in the memory 130. In case that the second object is an item, the processor 120 may measure the second temperature of the second object on the basis of a second algorithm stored in the memory 130. The electronic device 101 may identify the type of object corresponding to the preset gesture and measure the temperature of the identified object by applying an algorithm corresponding to the identified type of object.

The substrate (e.g., the substrate 240 in FIG. 3) may include a flexible substrate (flexible printed circuit board (FPCB)) having flexibility so as to correspond to a curvature of the electronic device 101 (e.g., an internal curvature corresponding to the opening 2001 of the housing 201).

The power management module (e.g., the power management module 188 in FIG. 1) may manage electric power supplied to at least one electric element included in the electronic device 101. For example, the power management module 188 may use a charging interface 213 to manage electric power supplied to the constituent elements from a battery (e.g., the battery 189 in FIG. 1). For example, the power management module 188 may include at least a part of a power management integrated circuit (PMIC).

The communication module (e.g., the communication module 190 in FIG. 1 or a communication circuit 390 in FIG. 3) may connect the electronic device 101 and an external electronic device (e.g., the electronic devices 102 and 104 in FIG. 1, a portable electronic device, a smartphone). For example, the communication module 190 (e.g., the communication circuit 390) may be electrically connected to an antenna module (e.g., the antenna module 197 in FIG. 1) and transmit signals or electric power to the external electronic devices 102 and 104 or receive signals or electric power from the external electronic devices 102 and 104 through the antenna module 197. For example, the antenna module 197 may include a conductor formed on the substrate 240 or a radiator configured by a conductive pattern. The electronic device 101 may receive a control signal from the external electronic devices 102 and 104 (e.g., a portable terminal device) through the communication module 190 and at least partially controlled in response to the control signal. For example, the electronic device 101 may at least partially activate the temperature sensors 210 and 211 in response to the control signal and provide the external electronic devices 102 and 104 with temperature information measured by the temperature sensors 210 and 211.

The electronic device 101 may include at least one of at least one display (not illustrated) (e.g., the display module 160 in FIG. 1) configured to provide the user with visual output information, an audio module (e.g., the audio module 170 in FIG. 1 or an audio circuit 370 in FIG. 3) configured to provide the user with auditory output information, and/or a haptic module (not illustrated) (e.g., the haptic module 179 in FIG. 1) configured to provide the user with tactile output information.

The processor 120 of the electronic device 101 may detect, periodically or in real time, a motion of the electronic device 101 by using the inertial sensor 220. For example, the processor 120 may identify whether the motion of the electronic device 101 is matched with the preset gesture and at least partially activate the temperature sensor 210 (e.g., the first temperature sensor 210) in response to the matching. In case that the detected motion is matched with a particular gesture, the electronic device 101 may automatically supply electric power to the temperature sensor 210 and at least partially activate the temperature sensor 210. The electronic device 101 may switch a deactivated state of the temperature sensor 210 to an activated state.

The processor 120 may identify the object corresponding to the preset gesture. For example, in case that the motion of the electronic device 101 is matched with the first gesture, the processor 120 may determine that the object is the first user (e.g., the wearer) corresponding to the first gesture. In case that the motion of the electronic device 101 is matched with the second gesture, the processor 120 may determine that the object is the second user (e.g., another person, an object, or an item) corresponding to the second gesture.

The processor 120 may measure the first temperature of the first object (e.g., the person) on the basis of the first algorithm stored in the memory 130 in response to the situation in which the object is the first object. The processor 120 may measure the second temperature of the second object (e.g., the item) on the basis of the second algorithm stored in the memory 130 in response to the situation in which the object is the second object. For example, in case that the object is a person, the first temperature of the object may be measured by applying the first algorithm. For example, in case that the object is an item, the second temperature of the object may be measured by applying the second algorithm.

In response to the situation in which the motion of the electronic device 101 is matched with the preset gesture, the electronic device 101 may at least partially activate the temperature sensor 210, and substantially simultaneously, the electronic device 101 may determine the type of object corresponding to the preset gesture. The electronic device 101 may apply the algorithm for measuring the temperature in accordance with the type of object and measure the temperature of the object. In response to at least one gesture matching, the electronic device 101 may activate the temperature sensor 210 and measure the temperature of the object corresponding to the gesture. The electronic device 101 may determine whether to activate the temperature sensor on the basis of the detected motion and supply electric power to the temperature sensor when the temperature sensor is activated. In the embodiment, it is possible to receive electric power consumption required to operate the temperature sensor. The efficiency of the battery 189 of the electronic device 101 may be improved.

FIG. 3 illustrates at least one constituent element (e.g., electric element) included in the electronic device 101. The electronic device 101 in FIG. 3 may include the processor 120 (e.g., the processor 120 in FIG. 2), the memory 130 (e.g., the memory 130 in FIG. 2), the audio circuit (audio circuitry) 370 (e.g., the audio module 170 in FIG. 1), the battery 189 (e.g., the battery 189 in FIG. 2), the communication circuit (communication circuitry) 390 (e.g., the communication module 190 in FIGS. 1 and 2), the temperature sensor 210 (e.g., the first temperature sensor 210 in FIG. 2), and/or the inertial sensor 220 (e.g., the inertial sensor 220 in FIG. 2). The temperature sensor 210 may include the contactless temperature sensor and defined as the first temperature sensor 210 illustrated in FIG. 2. Hereinafter, the temperature sensor 210 means the first temperature sensor 210 in FIG. 2 (e.g., the contactless temperature sensor). For example, the memory 130 may store a first algorithm 311 to be applied to the first object (e.g., the person) and a second algorithm 312 to be applied to the second object (e.g., the item).

The processor 120 of the electronic device 101 may detect, periodically or in real time, the motion of the electronic device 101 by using the inertial sensor 220 (e.g., a 3-axis sensor, a 6-axis sensor, an acceleration sensor, and a gyro sensor). For example, the processor 120 may identify whether the motion of the electronic device 101 is matched with the preset gesture (e.g., the first gesture, the second gesture, or the third gesture), and the processor 120 may at least partially activate the temperature sensor 210 in response to the matching. For example, in case that the detected motion is matched with a particular gesture, the processor 120 may automatically supply electric power to the temperature sensor 210 and at least partially activate the temperature sensor 210. The electronic device 101 may switch the deactivated state of the temperature sensor 210 to the activated state. The configuration in which the motion of the electronic device 101 is matched with the preset gesture (e.g., the first gesture, the second gesture, or the third gesture) may be defined as a configuration in which a first condition (e.g., a trigger condition for the temperature sensor 210) for activating the temperature sensor 210 is satisfied. The processor 120 may activate the temperature sensor 210 in response to the situation in which at least one gesture is matched among the preset gestures.

The processor 120 may identify the object corresponding to the detected motion. For example, in case that the motion of the electronic device 101 is matched with the first gesture, the processor 120 may determine that the object is the first object (e.g., the user, i.e., the wearer) corresponding to the first gesture. For example, in case that the motion of the electronic device 101 is matched with the second gesture, the processor 120 may determine that the object is the second object (e.g., another person or another user) corresponding to the second gesture. For example, in case that the motion of the electronic device 101 is matched with the third gesture, the processor 120 may determine that the object is the third object (e.g., an object or an item) corresponding to the third gesture. In response to the situation in which the object is the first object, the processor 120 may measure the first temperature of the first object on the basis of the first algorithm 311 stored in the memory 130. in response to the situation in which the object is the second object, the processor 120 may measure the second temperature of the second object on the basis of the second algorithm 312 stored in the memory 130.

In case that the motion of the electronic device 101 is matched with the preset gesture, the electronic device 101 may determine that the trigger condition (e.g., the first condition) for activating the temperature sensor 210 is satisfied.

In response to the situation in which the motion of the electronic device 101 is matched with the preset gesture (e.g., the first gesture, the second gesture, or the third gesture), the electronic device 101 may at least partially activate the temperature sensor 210, and substantially simultaneously, the electronic device 101 may determine the type of object (e.g., the user, i.e., the wearer, another person, an item, or an object) corresponding to the preset gesture. The electronic device 101 may apply the algorithm (e.g., the first algorithm 311 or the second algorithm 312) for measuring the temperature in accordance with the type of object and measure the temperature of the object. In response to at least one gesture matching, the electronic device 101 may activate the temperature sensor 210 and measure the temperature of the object corresponding to the matched gesture. According to the embodiment, the electronic device 101 may determine whether to activate the temperature sensor on the basis of the detected motion and supply electric power to the temperature sensor when the temperature sensor is activated. In the embodiment, it is possible to receive electric power consumption required to operate the temperature sensor. The efficiency of the battery 189 of the electronic device 101 may be improved.

The electronic device 101 may include the audio circuit 370 (e.g., the audio module 170 in FIG. 1) and use the audio circuit 370 to recognize the user's voice signal (e.g., an audio signal). The electronic device 101 may at least partially activate the temperature sensor 210 on the basis of the recognized audio signal. For example, in case that the user intends to arbitrarily activate the temperature sensor 210, the electronic device may at least partially activate the temperature sensor 210 by using the preset audio signal. The electronic device 101 may at least partially activate the temperature sensor 210 in response to the situation in which the recognized audio signal is matched with the preset voice signal.

The wearable electronic device (e.g., the electronic device 101 in FIGS. 1 to 3) may include the housing (e.g., the housing 201 in FIG. 2) including the first surface and the second surface positioned to be opposite to the first surface and configured to come into contact with a part of the body when the wearable electronic device is worn, the temperature sensor (e.g., the first temperature sensor 210 in FIG. 2 and the temperature sensor 210 in FIG. 3) disposed in the internal space of the housing 201 and configured to measure the temperature of the object adjacent to the first surface, the inertial sensor (e.g., the inertial sensor 220 in FIGS. 2 and 3) disposed in the internal space, the memory (e.g., the memory 130 in FIGS. 1 to 3), and the processor (e.g., the processor 120 in FIGS. 1 to 3) operatively connected to the temperature sensor 210, the inertial sensor 220, and the memory 130. The processor 120 may use the inertial sensor 220 to detect the motion of the wearable electronic device 101. When the first condition corresponding to the detected motion is satisfied, the processor 120 may activate the temperature sensor 210. The processor 120 may identify the object on the basis of the detected motion. The processor 120 may measure the temperature of the identified object by using the activated temperature sensor 210.

In the wearable electronic device 101, the housing 201 may be implemented in a ring shape. The wearable electronic device 101 may be worn in a state in which the second surface is at least partially in contact with a part of the body.

A part of the body may include the finger. The temperature sensor 210 may be disposed to be directed toward the palm in a closed fist and directed toward the outside in an open fist.

The processor 120 may identify a first value corresponding to the motion detected by the inertial sensor 220. In case that the first value is included in a preset threshold range, the processor 120 may at least partially activate the temperature sensor 210.

The processor 120 may use the inertial sensor 220 and identify the first gesture in which the wearable electronic device 101 moves to the forehead of the user wearing the wearable electronic device 101. The processor 120 may at least partially activate the temperature sensor 210 in response to the identification of the first gesture.

The processor 120 may use the inertial sensor 220 and identify the second gesture in which the wearable electronic device 101 moves to the object. The processor 120 may at least partially activate the temperature sensor 210 in response to the identification of the second gesture.

The temperature sensor 210 may include an infrared sensor configured to acquire infrared energy generated from the object.

In response to the situation in which the object is a person, the processor 120 may measure the first temperature of the object on the basis of the first algorithm 311 stored in the memory 130. in response to the situation in which the object is an item, the processor 120 may measure the second temperature of the object on the basis of the second algorithm 312 stored in the memory 130.

After measuring the temperature of the object, the processor 120 may use the inertial sensor 220 to detect the motion of the wearable electronic device 101. When a second condition corresponding to the detected motion is satisfied, the processor 120 may deactivate the temperature sensor 210 in the activated state.

The wearable electronic device 101 may further include the communication circuit (e.g., the communication circuit 390 in FIG. 3). The processor 120 may receive the control signal from the external electronic devices 102 and 104 operatively connected through the communication circuit 390. The processor 120 may measure the temperature of the object in response to the control signal.

The processor 120 may transmit the measured temperature to the external electronic devices 102 and 104 through the communication circuit 390. The processor 120 may display a user interface related to the measured temperature through the displays of the external electronic devices 102 and 104.

The wearable electronic device 101 may further include the audio circuit (e.g., the audio module 170 in FIG. 1 or the audio circuit 370 in FIG. 3). The processor 120 may acquire an audio signal through the audio circuit 370. The processor 120 may measure the temperature of the object by activating the temperature sensor 210 in response to the acquired audio signal.

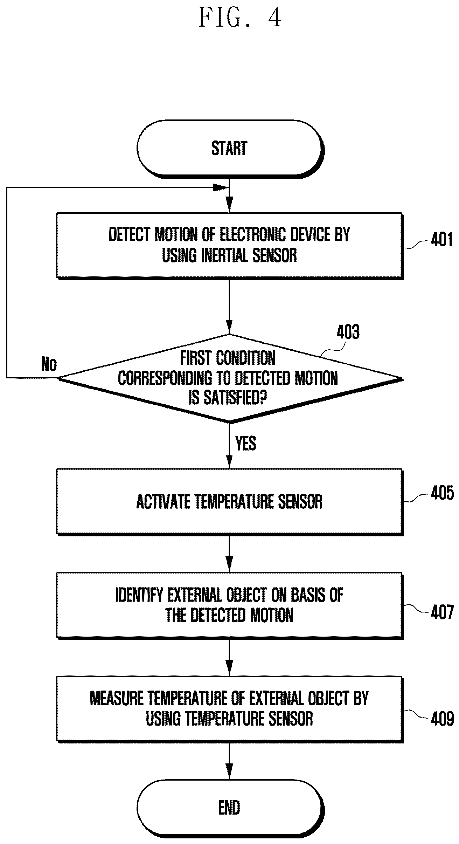

FIG. 4 is a flowchart illustrating a method of measuring the temperature of the object by the electronic device according to an embodiment of the disclosure.

Referring to FIG 4, the respective operations may be performed sequentially. However, the operations need not be necessarily performed sequentially. For example, the order of the respective operations may be changed, and at least two operations may be performed in parallel.

Operations 401 to 409 may be understood as being performed by the processor (e.g., the processor 120 in FIGS. 1 to 3) of the electronic device (e.g., the electronic device 101 in FIGS. 1 to 3).

The electronic device 101 in FIG. 4 may be at least partially similar to the electronic device 101 in FIGS. 1 to 3 or further include other embodiments of the electronic device. For example, the electronic device 101 may include a wearable electronic device capable of being worn on a part of a human body (e.g., a finger). The electronic device 101 may include the ring-type (e.g., ring-shape) wearable electronic device configured to be worn on the user's finger.

In operation 401, the electronic device 101 may detect the motion of the electronic device 101 by using the inertial sensor (e.g., the inertial sensor 220 in FIGS. 2 and 3) among the constituent members included in the housing (e.g., the housing 201 in FIG. 2). For example, the processor 120 may determine whether the motion of the electronic device 101 is matched with the preset gesture.

The electronic device 101 may measure the temperature of at least one object by using the temperature sensor (e.g., the first temperature sensor 210 in FIG. 2 and the temperature sensor 210 in FIG. 3) among the constituent members included in the housing 201. The temperature sensor 210 may include the contactless temperature sensor and defined as the first temperature sensor 210 illustrated in FIG. 2. The electronic device 101 may apply another algorithm depending on the type of object at the time of measuring the temperature of the object. For example, in case that the object is a person, the electronic device 101 may apply the first algorithm (e.g., the first algorithm 311 in FIG. 3) stored in the memory (e.g., the memory 130 in FIGS. 2 and 3). As another example, in case that the object is an item, the electronic device 101 may apply the second algorithm (e.g., the second algorithm 312 in FIG. 3) stored in the memory 130.

The processor 120 may use the inertial sensor 220 to detect the motion of the electronic device 101 and may at least partially activate the temperature sensor 210 in response to the situation in which the detected motion is matched with the preset gesture. For example, the condition in which the motion of the electronic device 101 is matched with the preset gesture may include the trigger condition for activating the temperature sensor 210.

The electronic device 101 may configure in advance the object corresponding to the preset gesture. For example, the processor 120 may configure in advance the first object (e.g., the user, i.e., the wearer) corresponding to the first gesture. The processor 120 may immediately identify the first object in response to the detection of the first gesture and measure the first temperature of the first object by applying the first algorithm 311 corresponding to the identified first object. As another example, the processor 120 may configure in advance the second object (e.g., the item) corresponding to the second gesture. The processor 120 may immediately identify the second object in response to the detection of the second gesture and measure the second temperature of the second object by applying the second algorithm 312 corresponding to the identified second object.

In response to the situation in which the motion of the electronic device 101 is matched with the preset gesture, the electronic device 101 may at least partially activate the temperature sensor 210 and identify the object corresponding to the matched gesture. The electronic device 101 may measure the temperature of the identified object by using the activated temperature sensor 210. In response to at least one gesture operation, the electronic device 101 may achieve the temperature sensor 210 and measure the temperature of the identified object. It is thus possible to improve the user's convenience and the efficiency in using electric power at the time of measuring the temperature by using the electronic device 101 (e.g., the wearable electronic device).

At operation 401, the processor 120 may use the inertial sensor 220 to detect the motion of the electronic device 101. For example, the inertial sensor 220 may include at least one of a 3-axis sensor, a 6-axis sensor, an acceleration sensor, and/or a gyro sensor. The processor 120 may use the inertial sensor 220 and identify coordinate information (e.g., gesture information or movement information) in accordance with a posture, a position, and a motion of the electronic device 101. For example, the processor 120 may detect a gesture of a motion of the finger on which the electronic device 101 is worn.

At operation 403, the processor 120 may determine whether the first condition (e.g., the condition for activating the temperature sensor 210) corresponding to the detected motion is satisfied. For example, the processor 120 may identify the first value (e.g., motion-related information or a motion-related coordinate value) corresponding to the detected motion and determine whether the identified first value is included in a preset threshold range. For example, the configuration in which the first value is included in the preset threshold range may be defined as a configuration in which the first condition is satisfied. At least one gesture (e.g., the first gesture, the second gesture, or the third gesture) according to the particular motion of the electronic device 101 may be configured in advance. For example, the situation in which the detected motion is matched with the preset gesture may include a situation in which the first condition is satisfied. The first gesture (e.g., a first threshold range based on the first gesture) in which the user touches his/her forehead with the palm in the state in which the electronic device 101 is worn on the finger may be configured, the second gesture (e.g., a second threshold range based on the second gesture) in which the user touches a forehead of another person (e.g., the counterpart) with the palm may be configured, and the third gesture (e.g., a third threshold range based on the third gesture) in which the user at least partially surrounds and grasps an item may be configured. When the processor 120 identifies that the detected motion is matched with one of the preset first gesture, the preset second gesture, and the preset third gesture, the processor 120 may determine that the first condition is satisfied. In case that the first value (e.g., the motion-related information or the motion-related coordinate value) corresponding to the detected motion is included in the preset threshold range (e.g., the first threshold range, the second threshold range, or the third threshold range), the processor 120 may determine that the first condition is satisfied, and the processor 120 may identify the corresponding gesture.

When the first condition is satisfied at operation 403, the processor 120 may activate the temperature sensor 210 at operation 405. For example, the temperature sensor 210 may be provided in the direction corresponding to the first surface (e.g., the outer surface) of the electronic device 101 and disposed in the internal space of the electronic device 101. For example, the temperature sensor 210 may be disposed in the direction in which the palm is directed in the state in which the electronic device 101 is worn on the finger, and the processor 120 may measure the temperature of the object in accordance with the palm direction by using the temperature sensor 210. When the first condition is not satisfied at operation 403, the electronic device 101 may return to operation 401 and perform the operation of detecting the motion of the electronic device 101 by using the inertial sensor 220. For example, the electronic device 101 may periodically or aperiodically and repeatedly perform the operation of determining whether the motion of the electronic device 101 satisfies the first condition according to operations 401 to 403.

At operation 407, the processor 120 may identify the external object on the basis of the detected motion. For example, in case that the detected motion is matched with the first gesture, the external object may be the user, i.e., the wearer. In case that the detected motion is matched with the second gesture, the external object may be another person (e.g., the counterpart). In case that the detected motion is matched with the third gesture, the external object may be an item or an object. The first gesture may be a gesture in which the user touches his/her forehead with the palm, the second gesture may be a gesture in which the user touches a forehead of another person with the palm, and the third gesture may be a gesture in which the user at least partially surrounds and grasps an item. The electronic device 101 may identify the type of external object in response to the situation in which the detected motion is matched with the preset gesture.

At operation 409, the processor 120 may use the temperature sensor 210 to measure the temperature of the external object. For example, in case that the detected motion is matched with the first gesture, the processor 120 may measure the first temperature of the user on the basis of the algorithm (e.g., the first algorithm 311) corresponding to the user. For example, in case that the detected motion is matched with the second gesture, the processor 120 may measure the second temperature of another person on the basis of the algorithm (e.g., the first algorithm 311) corresponding to another person (e.g., the counterpart). For example, in case that the detected motion is matched with the third gesture, the processor 120 may measure the third temperature of the item on the basis of the algorithm (e.g., the second algorithm 312) corresponding to the item.

In response to the situation in which the motion of the electronic device 101 is matched with the preset gesture (e.g., the first condition is satisfied), the electronic device 101 may at least partially activate the temperature sensor 210, and substantially simultaneously, identify the external object corresponding to the matched gesture. The electronic device 101 may use the at least partially activated temperature sensor 210 to measure the temperature of the identified object. In response to at least one gesture operation, the electronic device 101 may achieve the temperature sensor 210 and measure the temperature of the identified object, thereby improving the user's convenience.

The electronic device 101 may provide the user with a notification signal on the basis of the measured temperature. For example, in case that the first object is a person, a temperature of about 36.5 degrees, which is a normal body temperature, may be preset as a threshold value. In response to the situation in which the first temperature of the first object exceeds the threshold value (e.g., about 36.5 degrees), the processor 120 may provide the user with a notification signal. For example, the threshold value may vary depending on the types of objects. The electronic device 101 may provide the user with the notification signal in response to the situation in which the temperature of the object exceeds the preset threshold value. According to the embodiment, the electronic device 101 may output at least one effect among a visual effect, an auditory effect, or a tactile effect.

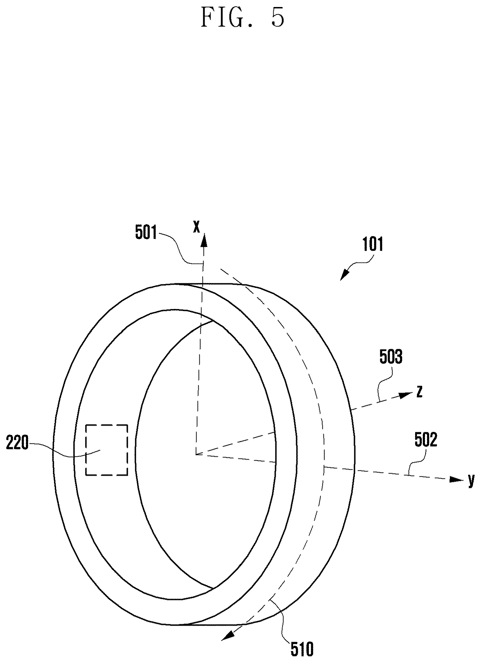

FIG. 5 is an exemplified view illustrating an embodiment in which the inertial sensor is used to identify the motion of the electronic device according to an embodiment of the disclosure.

Referring to FIG. 5, the electronic device 101 may be at least partially similar to the electronic device 101 in FIGS. 1 to 3 or further include other embodiments of the electronic device. For example, the electronic device 101 may include a wearable electronic device capable of being worn on a part of a human body (e.g., a finger). The electronic device 101 may include the ring-type (e.g., ring-shape) wearable electronic device configured to be worn on the user's finger.

The electronic device 101 may detect the motion of the electronic device 101 by using the inertial sensor (e.g., the inertial sensor 220 in FIGS. 2 and 3) among the constituent members included in the housing (e.g., the housing 201 in FIG. 2). For example, the inertial sensor 220 may include at least one of a 3-axis sensor, a 6-axis sensor, an acceleration sensor, and/or a gyro sensor.

Referring to FIG. 5, the processor 120 may use the inertial sensor 220 and detect at least one motion among a motion of the electronic device 101 in an x-axis direction 501, a motion in a y-axis direction 502, a motion in a z-axis direction 503, and/or a rotation motion in a first direction 510 (e.g., clockwise). The processor 120 may also detect a rotation motion of the electronic device 101 in a counterclockwise direction (not illustrated) opposite to the first direction 510. The processor 120 may individually divide the gestures into a gesture in which the user raises the hand on which the electronic device 101 is worn, a gesture in which the user touches the forehead with the hand, a gesture in which the user touches a forehead of another person with the hand, and/or a gesture in which the user surrounds and grasps the item with the hand. The processor 120 may detect the motion of the user's hand corresponding to the motion of the electronic device 101. The processor 120 may use the inertial sensor 220 and acquire a movement direction, a movement speed, and/or a movement time in accordance with the motion of the electronic device 101.

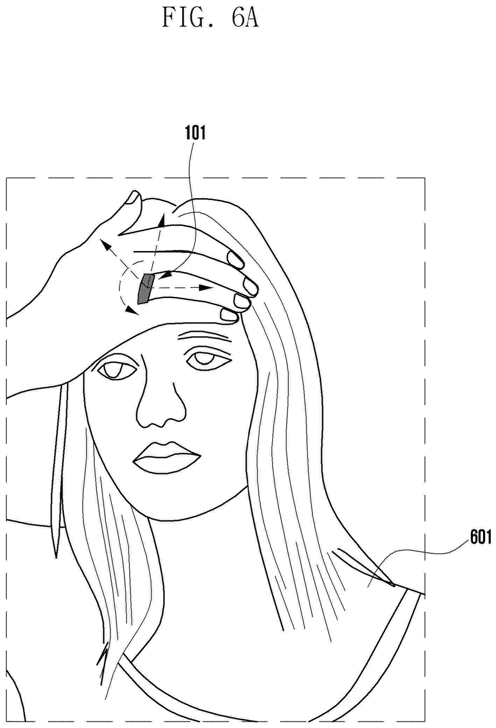

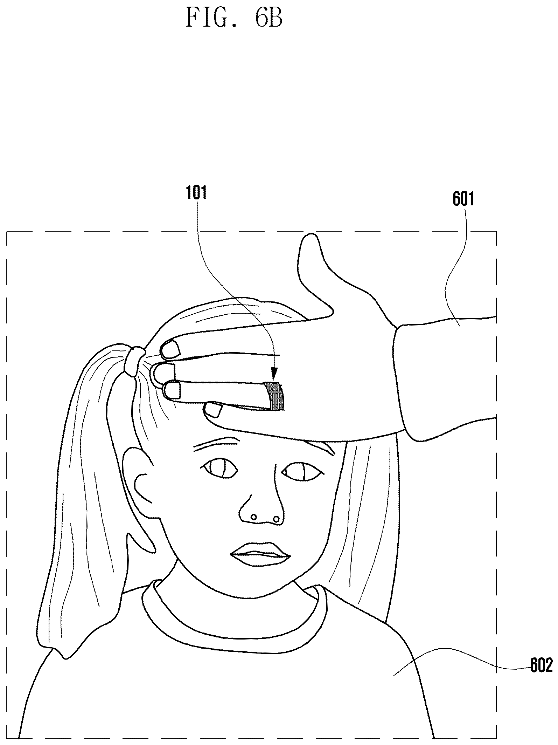

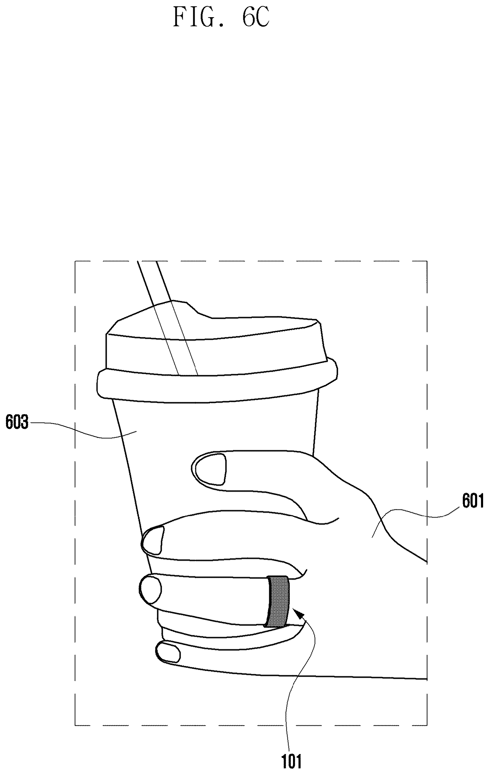

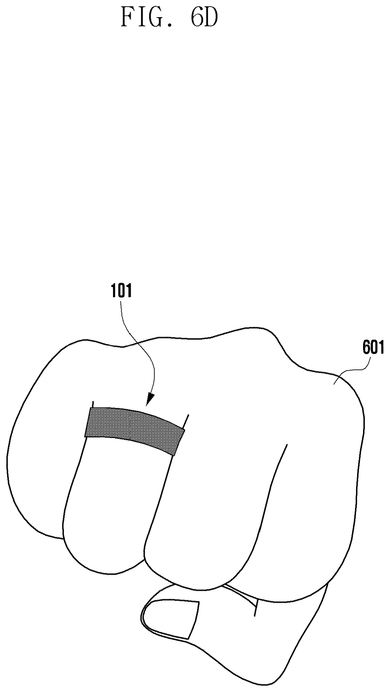

FIG. 6A is an exemplified view illustrating an embodiment in which the first temperature of the first object is measured in accordance with the first gesture according to an embodiment of the disclosure. FIG. 6B is an exemplified view illustrating an embodiment in which the second temperature of the second object is measured in accordance with the second gesture according to an embodiment of the disclosure. FIG. 6C is an exemplified view illustrating an embodiment in which the third temperature of the third object is measured in accordance with the third gesture according to an embodiment of the disclosure. FIG. 6D is an exemplified view illustrating an embodiment in which the preset function is performed in response to the detection of the preset gesture according to an embodiment of the disclosure.

Referring to FIGS. 6A to 6D, the electronic device 101 may be at least partially similar to the electronic device 101 in FIGS. 1 to 3 or further include other embodiments of the electronic device. For example, the electronic device 101 may include a wearable electronic device capable of being worn on a part of a human body (e.g., a finger). The electronic device 101 may include the ring-type (e.g., ring-shape) wearable electronic device configured to be worn on the finger of a user 601.

FIG. 6A illustrates the first gesture in which the user (e.g., the first object) touches the forehead in the state in which the user 601 wears the electronic device 101 on his/her finger. The electronic device 101 may detect the first gesture by using the inertial sensor (e.g., the inertial sensor 220 in FIGS. 2 and 3) included in the housing (e.g., the housing 201 in FIG. 2). For example, the first gesture may include a motion in which the user 601 directs the palm toward the inside (e.g., in a posture in which the palm is directed in the direction in which the user 601 is positioned) and the temperature sensor (e.g., the first temperature sensor 210 in FIG. 2 and the temperature sensor 210 in FIG. 3) included in the electronic device 101 moves to a position adjacent to the first object 601. The temperature sensor 210 may be the contactless temperature sensor that does not come into physical contact with the first object 601. The temperature sensor 210 may measure the first temperature of the first object 601 disposed within a preset distance.

The processor (e.g., the processor 120 in FIGS. 1 to 3) of the electronic device 101 may at least partially activate the temperature sensor 210 in response to the detection of the first gesture. The processor 120 may identify the first object (e.g., the user 601, i.e., the wearer) corresponding to the first gesture in response to the detection of the first gesture. The processor 120 may measure the temperature (e.g., the first temperature) of the user 601 by using the activated temperature sensor 210. The processor 120 may measure the first temperature of the user 601 by applying the algorithm (e.g., the first algorithm 311 in FIG. 3) related to the user 601 (e.g., the person). The processor 120 may store the measured first temperature in the memory (e.g., the memory 130 in FIGS. 1 to 3) and manage the measured first temperature. In response to the one detection of the gesture (e.g., the first gesture) of the electronic device 101, the temperature sensor 210 may be at least partially activated, and substantially simultaneously, the first temperature of the first object 601 may be measured by using the activated temperature sensor 210.

FIG. 6B illustrates the second gesture in which the user touches a forehead of a counterpart 602 (e.g., the second object or another person) in the state in which the user 601 wears the electronic device 101 on his/her finger. The electronic device 101 may use the inertial sensor 220 to detect the second gesture. For example, the second gesture may include a motion in which the user 601 directs the palm toward the outside (e.g., in a posture in which the palm is directed in a direction in which the user 601 is not positioned) and the temperature sensor 210 moves to a position adjacent to the second object 602. The temperature sensor 210 may be the contactless temperature sensor that does not come into physical contact with the second object 602. The temperature sensor 210 may measure the second temperature of the second object 602 disposed within a preset distance.

The processor 120 of the electronic device 101 may at least partially activate the temperature sensor 210 in response to the detection of the second gesture. The processor 120 may identify the second object (e.g., the counterpart 602 or another person) corresponding to the second gesture in response to the detection of the second gesture. The processor 120 may measure the temperature (e.g., the second temperature) of the counterpart 602 by using the activated temperature sensor 210. The processor 120 may measure the second temperature of the counterpart 602 by applying the algorithm (e.g., the first algorithm 311 in FIG. 3) related to the counterpart 602 (e.g., the person). The processor 120 may store the measured second temperature in the memory 130 and manage the measured second temperature. In response to the one detection of the gesture (e.g., the second gesture) of the electronic device 101, the temperature sensor 210 may be at least partially activated, and substantially simultaneously, the second temperature of the second object 602 may be measured by using the activated temperature sensor 210.

FIG. 6C illustrates the third gesture in which the user surrounds and grasps an object 603 (e.g., the third object or the item) in the state in which the user 601 wears the electronic device 101 on his/her finger. The electronic device 101 may use the inertial sensor 220 to detect the third gesture. For example, the third gesture may include a motion in which the palm is directed toward a surface of the object 603 and the temperature sensor 210 moves to a position adjacent to the object 603 (e.g., the third object). The temperature sensor 210 may be the contactless temperature sensor that does not come into physical contact with the third object 603. The temperature sensor 210 may measure the third temperature of the third object 603 positioned within a preset distance.

The processor 120 of the electronic device 101 may at least partially activate the temperature sensor 210 in response to the detection of the third gesture. The processor 120 may identify the third object (e.g., the object 603 or the item) corresponding to the third gesture in response to the detection of the third gesture. The processor 120 may measure the temperature (e.g., the third temperature) of the object 603 by using the activated temperature sensor 210. The processor 120 may measure the third temperature of the object 603 by applying the algorithm (e.g., the second algorithm 312 in FIG. 3) related to the object 603. The processor 120 may store the measured third temperature in the memory 130 and manage the measured third temperature. In response to the one detection of the gesture (e.g., the third gesture) of the electronic device 101, the temperature sensor 210 may be at least partially activated, and substantially simultaneously, the third temperature of the third object 603 may be measured by using the activated temperature sensor 210.

The electronic device 101 may be operatively connected to the external electronic device (e.g., the smartphone) through the communication circuit (e.g., the communication circuit 390 in FIG. 3). The processor 120 may provide the external electronic device with the temperature information (e.g., the first temperature, the second temperature, or the third temperature) of the identified object. For example, the external electronic device may individually manage the received temperature information and display a user interface (UI) related to the temperature information through a display. For example, when the first temperature (e.g., the temperature information of the user, i.e., the wearer) is provided, the external electronic device may display a first UI related to the user 601 (e.g., the first object) and output a notification signal on the basis of a threshold value (e.g., a normal body temperature or an average body temperature of the user 601) related to the user 601. As another example, when the second temperature (e.g., temperature information of another person) is provided, the external electronic device may display a second UI related to the counterpart 602 (e.g., the second object) and output a notification signal on the basis of a threshold value (e.g., an average body temperature of a general person or a universal average body temperature) related to the counterpart 602. In response to the reception of the second temperature (e.g., temperature information of another person), the external electronic device may display list information for selecting a particular counterpart or display a registration option (e.g., a registration icon) for registering a new counterpart. The temperature information related to the object corresponding to the preset gesture may be individually managed. The user's convenience may be improved during the process of measuring the temperature by using the electronic device 101 (e.g., the wearable electronic device).

The processor 120 may use the inertial sensor 220 and detect a situation in which the gesture (e.g., the first gesture, the second gesture, or the third gesture), which satisfies the first condition (e.g., the trigger condition for activating the temperature sensor 210), is released. In response to the situation in which at least one of the first gesture, the second gesture, and the third gesture is released, the processor 120 may at least partially deactivate the activated temperature sensor 210. In response to the detection of the particular gesture, the electronic device 101 may activate or deactivate the temperature sensor 210 and efficiently manage electric power consumed by the temperature sensor 210. The electronic device 101 may improve the efficiency of the battery and the user's usability.

FIG. 6D illustrates a fourth gesture in which the user 601 makes a fist in the state in which the electronic device 101 is worn on the finger of the user 601. According to the embodiment, the electronic device 101 may detect the fourth gesture by using at least one of the inertial sensor 220 and the temperature sensor 210.

The processor 120 of the electronic device 101 may at least partially activate the temperature sensor 210 in response to the detection of the fourth gesture. The processor 120 may identify the object (e.g., the user 601) corresponding to the fourth gesture in response to the detection of the fourth gesture (e.g., the gesture in which the user makes a fist). The processor 120 may measure the temperature (e.g., the first temperature) of the user 601 by using the activated temperature sensor 210. The processor 120 may measure the temperature of the user 601 by applying the algorithm (e.g., the first algorithm 311 in FIG. 3) related to the user 601.

The processor 120 may perform preset functions in response to the detection of the fourth gesture. For example, the processor 120 may perform functions (e.g., a near field communication (NFC) function or a Bluetooth (BT) function) related to the communication with the external electronic device in response to the detection of the fourth gesture. In response to the detection of the gesture (e.g., the fourth gesture) in which the user makes a fist, the processor 120 may activate a card payment function based on the NFC function.

The electronic device 101 may detect the motion of the electronic device 101 (e.g., the gesture) by using the temperature sensor 210 and the inertial sensor 220 and perform at least one preset function on the basis of the detected gesture. The electronic device 101 may detect the preset particular gesture by using the temperature sensor 210 and the inertial sensor 220 and perform at least one function corresponding to the detected particular gesture. For example, at least one function may be configured in advance so that the function is performed in response to the condition in which the particular gesture is detected. According to the embodiment, the electronic device 101 may automatically perform the preset particular function in response to at least one gesture operation. The user's convenience may be improved at the time of utilizing the preset particular function.

The electronic device 101 may further include the audio circuit (e.g., the audio module 170 in FIG. 1 or the audio circuit 370 in FIG. 3) and recognize the user's voice signal (e.g., an audio signal). The electronic device 101 may at least partially activate the temperature sensor 210 on the basis of the recognized audio signal. For example, in case that the user intends to arbitrarily activate the temperature sensor 210, the electronic device may at least partially activate the temperature sensor 210 by using the preset audio signal. The electronic device 101 may at least partially activate the temperature sensor 210 in response to the situation in which the recognized audio signal is matched with the preset voice signal.

The method of measuring the temperature by using the wearable electronic device (e.g., the electronic device 101 in FIGS. 1 to 3) according to the embodiment may include an operation of detecting the motion of the wearable electronic device 101 by using the inertial sensor (e.g., the inertial sensor 220 in FIGS. 2 and 3) disposed in the internal space of the housing (e.g., the housing 201 in FIG. 2), an operation of activating the temperature sensor (e.g., the first temperature sensor 210 in FIG. 2 and the temperature sensor 210 in FIG. 3) disposed in the internal space of the housing 201 in response to the situation in which the first condition corresponding to the detected motion is satisfied, an operation of identifying the object on the basis of the detected motion; and an operation of measuring the temperature of the identified object by using the activated temperature sensor 210.

According to the embodiment, the housing 201 may be implemented in a ring shape and include the first surface to which the object is adjacent, and the second surface positioned to be opposite to the first surface and configured to come into contact with a part of the body when the electronic device is worn.

According to the embodiment, the operation of activating the temperature sensor 210 may include an operation of identifying the first value corresponding to the detected motion by using the inertial sensor 220, and an operation of at least partially activating the temperature sensor 210 in case that the identified first value is included in the preset threshold range.

The method according to the embodiment may further include an operation of identifying, by using the inertial sensor 220, the first gesture in which the wearable electronic device 101 moves to the forehead of the user wearing the wearable electronic device 101, and an operation of at least partially activating the temperature sensor 210 in response to the identification of the first gesture.

The method of according to the embodiment may further include an operation of identifying, by using the inertial sensor 220, the second gesture in which the wearable electronic device 101 moves to the object, and an operation of at least partially activating the temperature sensor 210 in response to the identification of the second gesture.

According to the embodiment, the temperature sensor 210 may include an infrared sensor configured to acquire infrared energy generated from the object.

The method according to the embodiment may further include an operation of measuring the first temperature of the object on the basis of the first algorithm 311 stored in the memory 130 in response to the situation in which the identified object is a person, and an operation of measuring the second temperature of the object on the basis of the second algorithm 312 stored in the memory 130 in response to the situation in which the identified object is an item.

The method according to the embodiment may further include an operation of detecting the motion of the wearable electronic device 101 by using the inertial sensor 220 after the temperature of the object is measured, and an operation of deactivating the temperature sensor 210 in an activated state when the second condition corresponding to the detected motion is satisfied.

The electronic device according to various embodiments may be one of various types of electronic devices. The electronic devices may include, for example, a portable communication device (e.g., a smartphone), a computer device, a portable multimedia device, a portable medical device, a camera, a wearable device, a home appliance, or the like. According to an embodiment of the disclosure, the electronic devices are not limited to those described above.

It should be appreciated that various embodiments of the disclosure and the terms used therein are not intended to limit the technological features set forth herein to particular embodiments and include various changes, equivalents, or replacements for a corresponding embodiment. It is intended that features described with respect to separate embodiments, or features recited in separate claims, may be combined unless such a combination is explicitly specified as being excluded or such features are incompatible. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), the element may be coupled with the other element directly (e.g., wiredly), wirelessly, or via a third element.

As used in connection with various embodiments of the disclosure, the term "module" may include a unit implemented in hardware, software, or firmware, or any combination thereof, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the "non-transitory" storage medium is a tangible device, and may not include a signal (e.g., an electromagnetic wave), but this term does not differentiate between where data is semipermanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. A wearable electronic device comprising:
a housing including a first surface, and a second surface positioned to be opposite to the first surface and configured to come into contact with a part of a body when the wearable electronic device is worn;
a temperature sensor disposed in an internal space of the housing and configured to measure a temperature of an object adjacent to the first surface;
an inertial sensor disposed in the internal space;
memory storing one or more computer programs; and
one or more processors communicatively connected to the temperature sensor, the inertial sensor, and the memory,
wherein the one or more computer programs include computer-executable instructions that, when executed by the one or more processors, cause the wearable electronic device to:
detect a motion of the wearable electronic device by using the inertial sensor, and
when a first condition corresponding to the detected motion is satisfied, activate the temperature sensor, identify the object on a basis of the detected motion, and measure a temperature of the identified object by using the activated temperature sensor.

2. The wearable electronic device of claim 1, wherein the housing is implemented in a ring shape and worn in a state in which the second surface is at least partially in contact with a part of the body.

3. The wearable electronic device of claim 2,
wherein a part of the body includes a finger, and
wherein the temperature sensor is disposed to be directed to a palm in a closed fist and directed toward the outside in an open fist.

4. The wearable electronic device of claim 1, wherein the one or more computer programs further include computer-executable instructions that, when executed by the one or more processors, cause the wearable electronic device to:
identify a first value corresponding to the detected motion by using the inertial sensor, and
when the identified first value is included in a preset threshold range, at least partially activate the temperature sensor.

5. The wearable electronic device of claim 4, wherein the one or more computer programs further include computer-executable instructions that, when executed by the one or more processors, cause the wearable electronic device to:
identify, by using the inertial sensor, a first gesture in which the wearable electronic device moves to a forehead of a user wearing the wearable electronic device, and
at least partially activate the temperature sensor in response to the identification of the first gesture.

6. The wearable electronic device of claim 4, wherein the one or more computer programs further include computer-executable instructions that, when executed by the one or more processors, cause the wearable electronic device to:
identify, by using the inertial sensor, a second gesture in which the wearable electronic device moves to the object, and
at least partially activate the temperature sensor in response to the identification of the second gesture.

7. The wearable electronic device of claim 1, wherein the temperature sensor comprises an infrared sensor configured to acquire infrared energy generated from the object.

8. The wearable electronic device of claim 1, wherein the one or more computer programs further include computer-executable instructions that, when executed by the one or more processors, cause the wearable electronic device to:
measure a first temperature of the object on the basis of a first algorithm stored in the memory in response to a situation in which the identified object is a person, and
measure a second temperature of the object on the basis of a second algorithm stored in the memory in response to a situation in which the identified object is an item.

9. The wearable electronic device of claim 1, wherein the one or more computer programs further include computer-executable instructions that, when executed by the one or more processors, cause the wearable electronic device to:
detect a motion of the wearable electronic device by using the inertial sensor after a temperature of the object is measured, and
deactivate the temperature sensor in an activated state when a second condition corresponding to the detected motion is satisfied.

10. The wearable electronic device of claim 1, further comprising:
a communication circuit,
wherein the one or more computer programs further include computer-executable instructions that, when executed by the one or more processors, cause the wearable electronic device to:
receive a control signal from external electronic devices operatively connected through the communication circuit, and
measure a temperature of the object in response to the control signal.

11. The wearable electronic device of claim 10, wherein the one or more computer programs further include computer-executable instructions that, when executed by the one or more processors, cause the wearable electronic device to:
transmit the measured temperature to the external electronic devices through the communication circuit, and
display a user interface related to the measured temperature through displays of the external electronic devices .

12. The wearable electronic device of claim 1, further comprising:
an audio circuit,
wherein the one or more computer programs further include computer-executable instructions that, when executed by the one or more processors, cause the wearable electronic device to:
acquire an audio signal through the audio circuit, and
measure a temperature of the object by activating the temperature sensor on the basis of the acquired audio signal.

13. A method of measuring a temperature by using a wearable electronic device, the method comprising:
detecting a motion of the wearable electronic device by using an inertial sensor disposed in an internal space of a housing;
activating a temperature sensor disposed in the internal space of the housing in response to a situation in which a first condition corresponding to the detected motion is satisfied;
identifying an object on a basis of the detected motion; and
measuring a temperature of the identified object by using the activated temperature sensor.

14. The method of claim 13, wherein the activating of the temperature sensor comprises:
identifying a first value corresponding to the detected motion by using the inertial sensor; and
at least partially activating the temperature sensor when the identified first value is included in a preset threshold range.

15. The method of claim 13, further comprising:
measuring a first temperature of the object on the basis of a first algorithm stored in memory of the wearable device in response to a situation in which the identified object is a person; and
measuring a second temperature of the object on the basis of a second algorithm stored in the memory in response to a situation in which the identified object is an item.
